Europäisches Patentamt

European Patent Office

Office européen des brevets

(11) Veröffentlichungsnummer : **0 366 868 B1**

(12) **EUROPÄISCHE PATENTSCHRIFT**

(19)

(45) Veröffentlichungstag der Patentschrift :
**30.09.92 Patentblatt 92/40**

(51) Int. Cl.⁵ : **A61K 31/275,** A61K 9/22,
A61K 9/26

(21) Anmeldenummer : **89112885.2**

(22) Anmeldetag : **13.07.89**

(54) **Arzneimittel-Depotform auf Alginatbasis.**

(30) Priorität : **13.07.88 DE 3823693**

(43) Veröffentlichungstag der Anmeldung :
**09.05.90 Patentblatt 90/19**

(45) Bekanntmachung des Hinweises auf die
Patenterteilung :
**30.09.92 Patentblatt 92/40**

(84) Benannte Vertragsstaaten :
**ES**

(56) Entgegenhaltungen :
**EP-A- 0 063 266**
**GB-A- 2 207 353**
**ARZNEIM. -FORSCH. (DRUG RES.), Band 25,**
**Nr. 8, 1975; D.MAYER et al., Seiten, 1272-1275.**
**JOURNAL OF CONTROLLED RELEASE, band**
**3, Nr. 2/3, März 1986, Elsevier Science Publis-**
**hers B.V., Amsterdam (NL); A.F.STOCKWELL**
**etal., Seiten 167-175.**

(73) Patentinhaber : **KNOLL AG**
**Knollstrasse 32**
**W-6700 Ludwigshafen (DE)**

(72) Erfinder : **Balz, Evamarie**
**Naechstenbacher Weg 93**
**W-6940 Weinheim (DE)**
Erfinder : **Einig, Heinz, Dr.**
**Aspenweg 12**
**W-6730 Neustadt (DE)**
Erfinder : **Dresen, Peter, Dr.**
**Im Wingert 6 d**
**W-6806 Viernheim (DE)**

(74) Vertreter : **Höller, Klaus, Dr. et al**
**BASF Aktiengesellschaft Carl-Bosch-Strasse**
**38**
**W-6700 Ludwigshafen (DE)**

## Beschreibung

Die vorliegende Erfindung betrifft die Herstellung einer Verapamilenthaltenden Arzneimittel-Depotform auf Alginatbasis.

Es ist bereits bekannt, daß man Alginate zur Herstellung von Depot-Arzneimitteln verwenden kann (Pharm. Ind., 33, 296 (1971) und Arzneim.Forsch. (Drug Res.) 25, 1272 (1975)). Das in der Arzneiform enthaltene Alginat bildet in der Magen- bzw. Darmflüssigkeit durch Quellung eine Gelmatrix, die den Wirkstoff einschließt. Durch Diffusion und Abbau der Gelmatrix wird der Wirkstoff über einen Zeitraum von etwa 6 bis 10 Stunden aus der Arzneiform freigesetzt.

In den Depotformen auf Alginatbasis läßt sich die Wirkstoff-Freisetzung bislang nur durch erhebliche Variationen bei der verwendeten Alginatmenge, durch Variation des Herstellungsprozesses sowie durch Veränderung von Menge und Art der übrigen verwendeten Hilfsstoffe beeinflussen.

So führte einerseits die Verwendung unterschiedlicher Alginatmengen und damit ein geändertes Wirkstoff/Hilfsstoffverhältnis stets zu einer speziellen und differierenden Wirkstoff-Freisetzung aus der jeweiligen Depotform.

Mußte andererseits eine aus medizinischen und biopharmazeutischen Gründen geforderte und in eng begrenzten Spezifikationen festgelegte Wirkstoff-Freisetzung eingehalten werden, ergaben sich bisher regelmäßig große Probleme, da die obigen, in ihren genauen Wirkungen nur schwer überschau- und steuerbaren Einflußgrößen im großtechnischen Maßstab zumeist nur unvollkommen konstant gehalten werden konnten. Dies wurde insbesondere bei Anwendung gut diskriminierender in vitro-Testverfahren (z.B. Paddle-Test gemäß USP bei 50 rpm) deutlich und führte zu Produktionsunsicherheiten und konnte die Marktversorgung gefährden.

In der Praxis stellen sich insbesondere die folgenden Problemstellungen:

1. Bei einer vorgegebenen Größe, Form und Rezeptur des Arzneimittels (z.B. bezüglich der äußeren Form gute Patientencompliance gegeben) soll eine bewußte Variation der Wirkstoff-Freisetzung mit dem Ziel eines geänderten Wirkprofils möglich sein.

2. Trotz hochdosiertem Wirkstoffgehalt soll eine kleine Arzneiform resultieren und die Freisetzung auf jeden gewünschten Wert eingestellt werden können.

3. Bei einer gegebenen Rezeptur und äußeren Form sowie einem bereits definierten Herstellverfahren soll die Wirkstoff-Freisetzung eines Produktes in engen Grenzen über lange Zeit und von Charge zu Charge konstant gehalten werden.

Der Erfindung liegt die Aufgabe zugrunde, ein Verfahren zur Herstellung einer Verapamil-enthaltenden Arzneimittel-Depotform bereitzustellen, welches die genannten Problemstellungen löst, und das insbesondere erlaubt, eine Arzneimittel-Depotform zu erhalten, bei der die Geschwindigkeit der Freisetzung des Wirkstoffs sehr präzise und von Charge zu Charge wiederholbar eingestellt werden kann, wobei die Arzneimittel-Depotform die folgende in vitro Freisetzungscharakteristik aufweist: (a) Freisetzung nahezu nullter Ordnung und (b) Wirkstofffreisetzung in % Wirkstoff, gemessen als Verapamil-Säureadditionssalz, im Bereich von 60 bis 100 % innerhalb von 8 Stunden.

Diese Aufgabe wird gelöst mit einem Verfahren der genannten Art, das dadurch gekennzeichnet ist, daß man ein Alginat einsetzt, dessen Viskosität in zuvor genau definierten (d.h. der Aufgabenstellung angepaßten, engen Grenzen liegt, wobei die Viskosität ein Wert zwischen 30 und 500 mPa.s aufweist, der eine Schwankungsbreite von ± 10 mPa.s zeigen kann.

Nach einer bevorzugten Ausführungsform der Erfindung liegt der Wert zwischen 60 und 120. Nach einer weiteren bevorzugten Ausführungsform der Erfindung stellt man als Arzneimittel-Depotform eine Oblong-Filmtablette her.

Als Wirkstoff liegt Verapamil bevorzugt als Säureadditionssalz, insbesondere als Hydrochlorid vor. Zweckmäßig liegt in der Arzneimittel-Depotform ein Gewichtsverhältnis Verapamil-HCl:Alginat von 1:0,7 bis 1:3 vor.

Gegenstand der Erfindung sind somit Verfahren zur Herstellung verschiedener Verapamil-haltigen Depotformen auf Alginatbasis, bei denen bei Einsatz konstanter (Problemfall 1. und 3.) oder unterschiedlicher Alginatmengen (Problemfall 2.) eine spezielle, therapeutisch geforderte Geschwindigkeit der Wirkstoff-Freisetzung erreicht wird.

Das in den Rezepturen verarbeitete Alginat ist dadurch gekennzeichnet, daß seine Viskosität in einer 1 %igen wäßrigen Lösung einen Wert zwischen 30 und 500 aufweist, wobei dieser Wert eine Schwankungsbreite von ± 10 mPa.s zeigen kann.

Die Viskosität des Alginats muß innerhalb eines engen Bereiches von ≤20 mPa·s liegen. Je enger der Bereich der Viskosität liegt, desto enger begrenzt sind auch die Liberationseigenschaften der Arzneiform.

Der beanspruchte Viskositätsbereich bezieht sich auf die Messung der Viskosität mit einem Hake-Viskosimeter (Modell Rheomat® 30) bzw. mit einem Brookfield-Viskosimeter (Modell DV2). Mißt man die Viskosität mit anderen Geräten, so kann sich der Viskositätsbereich verschieben. Weiter ist zu beachten, daß bei der Herstellung der 1 %igen wäßrigen Alginatlösung für die Bestimmung der Viskosität der 1 %ige Alginatanteil auf getrocknetes Alginat berechnet wird.

Die Herstellung der Alginate ist bekannt (vgl. Ullmanns Encyklopädie der technischen Chemie, 4. Auflage, Band 19, Seiten 245 bis 246 und darin zitierte Literatur; W.H. McNeely, D.J. Pettitt in Whistler: Industrial Gums, 2. Auflage, Academic Press, New York, Seite 49 ff).

Bevorzugt verwendet man solche Alginate, in denen das Verhältnis von Manuron- zu Guluronsäure zwischen 7:3 und 3:7, vorzugsweise zwischen 6:4 und 4:6, liegt. Diese lassen sich z.B. aus der Alge Laminaria hyperborea gewinnen.

Als Alginate kommen beispielsweise Ammoniumalginat, Kaliumalginat, Natriumalginat oder Mischungen aus diesen Alginaten in Betracht. Bevorzugt ist Natriumalginat.

Je höher das Molekulargewicht (Viskosität) des Alginats ist, desto niedriger ist die Freisetzungsgeschwindigkeit des Wirkstoffs unter der Voraussetzung, daß die Alginatmenge in der Rezeptur konstant gehalten wird. Je enger der Bereich der Viskosität begrenzt wird, desto weniger streuen die Werte der Freisetzung von Charge zu Charge.

Die Anwendung der Erfindung ermöglicht insbesondere die problemlose Herstellung von auf Alginatbasis beruhenden Verapamil-haltigen Depot-Arzneimitteln, die einer aus medizinischer Sicht geforderten, eng begrenzten Wirkstoff-Freisetzung entsprechen. Insbesondere wird damit ein hoher Grad an Produktionssicherheit erreicht, und es kann durch Vermeiden von Fehlchargen eine besonders wirtschaftliche Produktionsweise gewährleistet werden.

Beispiel 1, Herstellung von Alginaten

Ein trockenes Gemisch aus Braunalgen, welches aus gleichen Teilen Laminaria spec., Ascofyllum nodosum, Macrocystis spec., Lesonia spec., Ecklonia spec. und Durvillea spec. bestand, wurde auf einer Schnitzelmaschine grob zerkleinert, so daß Stücke mit einer Länge von bis zu 3 cm entstanden. Das so erhaltene Material wurde 3 x wechselweise mit heißem Wasser und 0,02 N Salzsäure zur Entfernung säurelöslicher Bestandteile gewaschen. 50 kg so erhaltene Feuchtmasse wurden mit 2000 l 0,02 N Natronlauge 4 h bei Raumtemperatur intensiv gerührt. Nach Sedimentation wurde die überstehende Flüssigkeit (Rohalginatlösung) abgesaugt. Zu der abgetrennten Flüssigkeit wurde so lange Calciumchlorid zugesetzt, bis das Rohalginat praktisch vollständig ausgefällt war. Der Niederschlag wurde abfiltriert und mit 1 N Salzsäure versetzt, bis der pH-Wert der Aufschlämmung bei pH 1 bis 2 lag. Der verbleibende Niederschlag (= Alginsäure) wurde abgetrennt und so lange mit kaltem Wasser gewaschen, bis der Calciumgehalt des Niederschlags unter 0,3 % (bezogen auf Trockenmasse) lag. Anschließend wurde die noch feuchte Alginsäure durch Anteigen mit Natriumcarbonat neutralisiert, wobei unter Wärmeentwicklung eine Paste entstand. Nachdem sich die Paste auf Raumtemperatur abgekühlt hatte, wurde sie in einem Drehrohrofen etwa 8 h bei einer Temperatur von 70°C und 4 Umdrehungen pro min getrocknet. Man erhielt 9,9 kg Natriumalginat mit einer Viskosität von etwa 180 mPa·s.

Durch Variation des Temperaturbereichs bei der Trocknung im Drehrohrofen von 50 bis 90°C kann man Produkte mit dem Viskositätsbereich von 30 bis 500 mPa·s erhalten.

Durch Mahlung wurde die gewünschte Kornverteilung (98 % < 50 µm) erreicht. Durch diesen Prozeß wird eine leichte Viskositätserniedrigung verursacht. Durch Mischen von Natriumalginaten verschiedener Viskositäten läßt sich gegebenenfalls eine gewünschte Viskosität einstellen. Die zu mischenden Alginate dürfen in ihren Viskositäten jedoch nicht mehr als etwa 60 mPa·s auseinanderliegen.

Beispiel 2, Herstellung von Arzneimitteln

Es wurden Depot-Arzneimittel (Filmtabletten) folgender Zusammensetzungen hergestellt:
Rezepturvariante 1 (220 mg Natriumalginat)

```
Verapamil-Hydrochlorid      240 mg
Natriumalginat              220 mg
Hilfsstoffe                 205 mg
                            665 mg Gesamtgewicht der Oblong-Filmtablette
```

Subvariante 1.1 mit Natriumalginat der Viskosität 204 mPa.s
Subvariante 1.2 mit Natriumalginat der Viskosität 348 mPa.s

Subvariante 1.3 mit Natriumalginat der Viskosität 501 mPa.s
Rezepturvariante 2 (320 mg Natriumalginat)

```
Verapamil-Hydrochlorid        240 mg
Natriumalginat                320 mg
Hilfsstoffe                   190 mg
                              750 mg Gesamtgewicht der Oblong-Filmtablette
```

Subvariante 2.1 mit Natriumalginat der Viskosität 87 mPa.s
Subvariante 2.2 mit Natriumalginat der Viskosität 143 mPa.s
Subvariante 2.3 mit Natriumalginat der Viskosität 204 mPa.s
Rezepturvariante 3 (400 mg Natriumalginat)

```
Verapamil-Hydrochlorid        240 mg
Natriumalginat                400 mg
Hilfsstoffe                   120 mg
                              760 mg Gesamtgewicht der Oblong-Filmtablette
```

Subvariante 3.1 mit Natriumalginat der Viskosität 30 mPa.s
Subvariante 3.2 mit Natriumalginat der Viskosität 62 mPa.s
Subvariante 3.3 mit Natriumalginat der Viskosität 87 mPa.s

Als Hilfsstoffe zur Herstellung der Tablettenmasse (Kern) wurden die folgenden Substanzen in folgendem Mengenverhältnis eingesetzt:

```
Mikrokristalline Cellulose:     40   bis 55 %
Povidone®:                      25   bis 35 %
Gereinigtes Wasser:             15   bis 25 %
Magnesiumstearat:              0,5 bis  2 %
```

Anstelle von Cellulose können auch Lactose, andere Zucker oder Stärken eingesetzt werden. Als Bindemittel ist ebenso Stärkekleister oder Gelatinelösung verwendbar. Ebenso können übliche Fließ- und Gleitmittel Verwendung finden.

Da Verapamil-HCl eine sehr bittere und anästhesierende Substanz ist, wurden die Tablettenkerne mit einem Filmlack geschmacksisoliert. Der eingesetzte Lack hatte folgende Zusammensetzung:

```
Hydroxypropylmethylcellulose    4,9 mg
Polyethylenglykol 400           1,3 mg
Polyethylenglykol 8000          0,8 mg
Talkum                          8,4 mg
Titandioxid                     6,2 mg
Farbstoffe                      0,1 mg
Wachs                           0,3 mg
```

Bei der Bestimmung der Wirkstoff-Freisetzung mit künstlichem Magen- und Darmsaft (Paddle-Test gemäß DAB 9 und USP XXI, 50 rpm, 900 ml, 37°C, Wechsel des Prüfmediums von pH 1,2 zu 7,4 nach der 1. Stunde [single change]) wurde die folgende Wirkstoff-Freisetzung ermittelt:

Bei allen Rezepturvarianten verlief die Wirkstoff-Freisetzung nahezu linear (Freisetzung 0. Ordnung), d.h. pH unabhängig. Nach 8 Stunden Testzeit war der Wirkstoff aus den Tabletten wie folgt freigesetzt:

| Variante | Wirkstoff-Freisetzung, $\bar{X}$ 6, VK < 5 % Verapamil-HCl in % |
|---|---|
| 1.1 | 94 |
| 1.2 | 83 |
| 1.3 | 70 |
| 2.1 | 90 |
| 2.2 | 81 |
| 2.3 | 72 |
| 3.1 | 92 |
| 3.2 | 79 |
| 3.3 | 69 |

Die bei den obigen Rezepturvarianten ermittelten Freisetzungswerte zeigen, daß auch bei Verwendung unterschiedlicher Alginatmengen durch gezielte Wahl des jeweils geeigneten Alginattyps jede gewünschte Wirkstoff-Freisetzung erreicht werden kann. Die Anwendung dieses Verfahrens erlaubt erstmalig eine sehr sichere und problemlose Herstellung von Verapamil-Depot-Arzneimittelformen auf Alginatbasis.

## Patentansprüche

1. Verfahren zur Herstellung von Verapamil-enthaltenden Arzneimittel-Depotformen auf Alginatbasis, die folgende in vitro Freisetzungscharakteristik aufweisen: (a) Freisetzung nahezu nullter Ordnung und (b) Wirkstoff-Freisetzung in % Wirkstoff, gemessen als Verapamil-Säure-additionssalz im Bereich von 60 bis 100 % innerhalb von 8 Stunden, wobei die Geschinwindigkeit der Freisetzung des Wirkstoffes sehr präzise und von Charge zur Charge wiederholbar aufgrund der Viskosität (in wäßriger Lösung) des verwendeten Alginats eingestellt wird und man ein Alginat einsetzt, dessen Viskosität in einer 1 %igen wäßrigen Lösung bei 20°C einen Wert aufweist, der zwischen 30 und 500 mPa.s liegt, wobei dieser Wert eine Schwankungsbreite von ± 10 mPa.s zeigen kann.

2. Verfahren nach Anspruch 1, dadurch gekennzeichnet, daß man ein Alginat einsetzt, dessen Viskosität in einer 1%igen wäßrigen Lösung bei 20°C einen Wert aufweist, der zwischen 60 und 120 MPa.s liegt, wobei dieser Wert eine Schwankungsbreite von ± 10 mPa.s zeigen kann.

3. Verfahren nach einem der Ansprüche 1 oder 2, dadurch gekennzeichnet, daß die Arzneimittel-Depotform als Oblong-Filmtablette hergestellt wird.

4. Verfahren nach einem der Ansprüche 1 bis 3, dadurch gekennzeichnet, daß in der Arzneimittel-Depotform ein Gewichtsverhältnis von Verapamil-HCl:Alginat von 1:0,7 bis 1:3 vorliegt.

## Claims

1. A process for producing alginate-based verapamil-containing pharmaceutical depot forms which have the following in vitro release characteristics: (a) virtually zero order release and (b) release of active substance in % active substance measured as verapamil acid addition salt in the range from 60 to 100% within 8 hours, the rate of release of the active substance being adjusted very precisely and repeatably from batch to batch on the basis of the viscosity (in aqueous solution) of the alginate used, and employing an alginate whose viscosity in a 1% strength aqueous solution at 20°C has a value which is from 30 to 500 mPa.s, it being possible for the range of variation of this value to be ± 10 mPa.s.

2. A process as claimed in claim 1, wherein an alginate whose viscosity in a 1% strength aqueous solution at 20°C has a value which is from 60 to 120 mPa.s, it being possible for the range of variation of this value to be ± 10 mPa.s, is employed.

3. A process as claimed in either of claims 1 or 2, wherein the pharmaceutical depot form is produced as oblong film-coated tablet.

4. A process as claimed in any of claims 1 to 3, wherein the verapamil hydrochloride:alginate ratio in the pharmaceutical depot form is from 1:0.7 to 1:3 by weight.

## Revendications

1. Procédé de préparation de médicaments à effet retard à base d'alginate, contenant du Verapamil, qui présentent la caractéristique de libération in vitro suivante :
   (a) libération d'ordre presque nulle et
   (b) libération du principe actif en % de principe actif, mesuré en tant que sel d'addition d'acide de Verapamil, dans les limites de 60 à 100 %, dans un délai de 8 heures, la vitesse de la libération du principe actif étant réglée très précise et répétable d'une charge à l'autre, sur la base de la viscosité (en solution aqueuse) de l'alginate utilisé et en employant un alginate dont la viscosité, dans une solution aqueuse à 1 %, présente, à 20 degrés C, une valeur comprise entre 30 et 500 mPa.s, cette valeur pouvant présenter une amplitude d'oscillation de ± 10 mPa.s.

2. Procédé selon la revendication 1, caractérisé en ce que l'on utilise un alginate dont la viscosité, dans une solution aqueuse à 1 %, présente, à 20 degrés C, une valeur qui est comprise entre 60 et 120 mPa.s cette valeur pouvant présenter une amplitude d'oscillation de ± 10 mPa.s.

3. Procédé selon l'une des revendication 1 ou 2 caractérisé en ce que le médicaments à effet retard est préparé sous forme de tablette mince oblongue.

4. Procédé selon l'une des revendications 1 à 3 caractérisé en ce que le médicament à effet retard présente un rapport en poids, chlorure de Verapamil/alginate, de 1/0,7 à 1/3.